(19) Europäisches Patentamt
European Patent Office
Office européen des brevets

(11) **EP 2 183 384 B1**

(12) **EUROPÄISCHE PATENTSCHRIFT**

(45) Veröffentlichungstag und Bekanntmachung des Hinweises auf die Patenterteilung:
**16.03.2016 Patentblatt 2016/11**

(21) Anmeldenummer: **08785201.8**

(22) Anmeldetag: **30.07.2008**

(51) Int Cl.:
**C12Q 1/32** (2006.01)  **C12Q 1/54** (2006.01)

(86) Internationale Anmeldenummer:
**PCT/EP2008/006255**

(87) Internationale Veröffentlichungsnummer:
**WO 2009/015870 (05.02.2009 Gazette 2009/06)**

(54) **VERFAHREN UND VORRICHTUNG ZUR BESTIMMUNG DER KONZENTRATION EINES ANALYTEN MITTELS FLUORESZENZMESSUNG**

METHOD AND DEVICE FOR DETERMINING THE CONCENTRATION OF AN ANALYTE USING MEASUREMENT OF FLUORESCENCE

PROCÉDÉ ET DISPOSITIF DESTINÉS À LA DÉTERMINATION DE LA CONCENTRATION D'UN ANALYTE PAR MESURE DE LA FLUORESCENCE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **01.08.2007 EP 07015084**

(43) Veröffentlichungstag der Anmeldung:
**12.05.2010 Patentblatt 2010/19**

(73) Patentinhaber:
• **Roche Diagnostics GmbH**
**68305 Mannheim (DE)**
Benannte Vertragsstaaten:
**DE**
• **F. Hoffmann-La Roche AG**
**4070 Basel (CH)**
Benannte Vertragsstaaten:
**AT BE BG CH CY CZ DK EE ES FI FR GB GR HR HU IE IS IT LI LT LU LV MC MT NL NO PL PT RO SE SI SK TR**

(72) Erfinder:
• **PETRICH, Wolfgang**
**76669 Bad Schönborn (DE)**
• **HAAR, Hans-Peter**
**69168 Wiesloch (DE)**
• **HOENES, Joachim**
**64673 Zwingenberg (DE)**
• **HORN, Carina**
**68647 Biblis (DE)**

(74) Vertreter: **Durm & Partner**
**Patentanwälte**
**Moltkestrasse 45**
**76133 Karlsruhe (DE)**

(56) Entgegenhaltungen:
WO-A-94/00602   WO-A-03/097859
WO-A-2004/044557   WO-A-2007/012494
US-A- 5 485 530   US-B2- 6 922 578

• SLAMA J T ET AL: "CARBANICOTINAMIDE ADENINE DINUCLEOTIDE SYNTHESIS AND ENZYMOLOGICAL PROPERTIES OF A CARBOCYCLIC ANALOGUE OF OXIDIZED NICOTINAMIDE ADENINE DINUCLEOTIDE" 1. Januar 1988 (1988-01-01), BIOCHEMISTRY, AMERICAN CHEMICAL SOCIETY, PAGE(S) 183 - 193 , XP002405653 ISSN: 0006-2960 Seite 192, linke Spalte, Absatz 4
• EVANS N D ET AL: "Glucose-dependent changes in NAD(P)H-related fluorescence lifetime of adipocytes and fibroblasts in vitro: Potential for non-invasive glucose sensing in diabetes mellitus" 1. August 2005 (2005-08-01), JOURNAL OF PHOTOCHEMISTRY AND PHOTOBIOLOGY B: BIOLOGY, ELSEVIER SCIENCE S.A., BASEL, CH, PAGE(S) 122-129 , XP004984395 ISSN: 1011-1344 das ganze Dokument Seite 125, rechte Spalte, Absatz 2 Seite 128, linke Spalte, Absatz 4 Abbildung 2

**Beschreibung**

Die Erfindung betrifft ein Verfahren zur Bestimmung der Konzentration von Glucose in einer flüssigen Probe. Insbesondere geht es um die Untersuchung medizinischer Proben, vor allem von Blut und/oder interstitieller Flüssigkeit auf darin enthaltene Analyten.

Speziell richtet sich die Erfindung auf Verfahren, bei denen die Ermittlung der Konzentration des Analyten auf einer Fluoreszenzmessung basiert. Bei solchen Verfahren wird ein Reagenzsystem verwendet, dessen Reaktion mit der Probe zu einer Änderung der Menge eines Fluorophors führt, wobei die Mengenänderung grundsätzlich sowohl eine Zunahme durch Bildung eines Fluorophors, als auch eine Abnahme durch Verbrauch eines Fluorophors sein kann. Das Reaktionssystem ist dabei so gewählt, dass die Änderung der Menge des Fluorophors für die gewünschte analytische Konzentration charakteristisch ist. Das Analyseverfahren schließt die Messung einer mit der Menge des Fluorophors korrelierenden Messgröße ein. Auf Basis der resultierenden Messwerte wird mittels eines Auswertealgorithmus die Konzentration ermittelt.

Derartige Verfahren sind bekannt, wobei beispielsweise auf folgende Dokumente verwiesen werden kann:

(1) EP 0 293 732 A2
(2) US 2005/0214891 A1
(3) US 2006/0003397 A1

Alle diese Publikationen beschreiben ein ähnliches Testprotokoll, bei dem das Reagenzsystem das Enzym-Coenzym-Paar Glucosedehydrogenase (GlucDH) / Nicotinamid-Adenin-Dinucleotid (NAD) enthält. Bei der Reaktion mit diesem Reagenzsystem wird unter dem Einfluss der GlucDH ein Hydridion aus der Glucose abgespalten und auf das NAD übertragen, so dass sich NADH bildet. Die gebildete Menge NADH ist charakteristisch für die Glucosekonzentration. NADH ist ein starker Fluorophor, dessen Konzentration durch eine Messung der Fluoreszenzintensität bestimmt werden kann. Die Korrelation der gemessenen Fluoreszenzintensität mit der Analytkonzentration wird üblicherweise durch Kalibration bestimmt. NADH ist auch ein Farbstoff. Deshalb kann es sinnvoll sein, ergänzend eine photometrische Messung (beispielsweise Messung der optischen Absorption bei einer bestimmten Wellenlänge) durchzuführen.

Diese Verfahrensweise ist grundsätzlich schon seit langem bekannt. Beispielsweise wird in dem Dokument (2) auf eine Publikation von Narayanaswamy et al. aus dem Jahr 1988 verwiesen, in der eine Fluoreszenzmessung mit GlucDH und NAD zur Glucosebestimmung verwendet worden sei. Mit der Messung der Fluoreszenzintensität ist jedoch eine Reihe von grundlegenden Problemen verbunden, die beispielsweise in dem Buch

(4) J.R. Lakovicz "Principles of Fluorescence Spectroscopy" Springer Science and Business Media, 2006

diskutiert werden:

- Die Intensität des detektierten Fluoreszenzsignals hängt von mehreren instrumentenspezifischen Faktoren ab, wie beispielsweise der Leistung der Lichtquelle, der Transmission der optischen Komponenten im Lichtweg oder der Sensitivität des Detektors. Unkontrollierte Änderungen dieser Faktoren beeinträchtigen die Messgenauigkeit.

- Eine weitere Fehlerquelle bei Intensitätsmessungen resultiert aus nicht spezifischem Licht, das aus der Umgebung an den Detektor dringt und eine unkontrollierte Signaländerung bewirken kann.

- Die Intensität des gemessenen Fluoreszenzlichts ist nicht nur von der Menge des Fluorophors abhängig, sondern wird auch von dessen molekularer Umgebung in der Probe erheblich beeinflusst. Dazu tragen insbesondere Prozesse bei, die unter dem Begriff Fluoreszenzlöschung ("quenching") zusammengefasst werden.

- Da zwischen der Anregung eines Moleküls und der Abstrahlung eines Lichtquants im statistischen Mittel eine Zeit in der Größenordnung von Nanosekunden vergeht, kann sich die Position und Orientierung des Moleküls zwischen Absorption und Emission ändern. Daraus resultieren Störeinflüsse hinsichtlich der Fluoreszenzintensität, insbesondere eine Temperaturabhängigkeit.

- Häufig erfolgt die Anregung der Fluoreszenz durch ultraviolettes Licht. Dabei können photochemische Reaktionen des elektronisch angeregten Zustandes zu einem Ausbleichen des Fluorophors ("bleaching") führen. Dies ist eine weitere Fehlerquelle.

Eine Verminderung dieser Probleme lässt sich dadurch erreichen, dass eine Kalibration der Messung der Fluoreszenzintensität mit Hilfe eines Fluorophors, dessen Fluoreszenzintensität bekannt ist, durchgeführt wird. Dies ist jedoch sehr aufwendig und eignet sich nur für anspruchsvolle Labormessungen.

Auf dieser Grundlage liegt der Erfindung das technische Problem zugrunde, ein Verfahren vorzuschlagen, das eine verbesserte Messgenauigkeit, insbesondere hinsichtlich der erwähnten Messfehler und Störungen mit vermindertem Aufwand ermöglicht. Es soll sich insbesondere auch für Kleingeräte eignen, die möglichst einfach und kostengünstig arbeiten.

Dieses technische Problem wird gelöst durch ein Verfahren nach Anspruch 1. Gegenstand der Erfindung ist auch ein Analysesystem zur Durchführung des Verfahrens.

Die Möglichkeit, Änderungen der Fluoreszenzlebensdauer für analytische Zwecke zu verwenden, wird in der WO 94/00602 im Zusammenhang mit einer in vivo-Analyse erwähnt. Soweit dort auf ein Verfahren zur Analyse von Glucose Bezug genommen wird, basiert dieses Verfahren auf zwei Reaktionen, nämlich einer ersten Reaktion, bei der die Glucose in Gegenwart von Glucoseoxidase unter Bildung von Wasserstoffperoxid reagiert und einer zweiten Reaktion, bei der das Wasserstoffperoxid mittels eines Leucofarbstoffes bestimmt wird.

Im Rahmen der Erfindung wurde festgestellt, dass bei Analysesystemen, bei denen die Reagenzien des Reagenzsystems in trockener Form in einem Analyseelement integriert sind, die Fluoreszenzlebensdauer überraschenderweise in einem so starken Ausmaß von der Analytkonzentration abhängt, dass Messwerte dieser Messgröße vorteilhaft in dem Algorithmus zur Ermittlung des analytischen Ergebnisses (gesuchte Konzentration) verwendet werden können. In Kenntnis der Erfindung lässt sich dies für den Fall eines Glucosetests mit dem Reagenzsystem GlucDH/NAD folgendermaßen erklären.

Das infolge der Reaktion der Glucose mit dem Enzym-Coenzym-Paar GlucDH/NAD gebildete NADH bildet mit der GlucDH einen Komplex. Diese Komplexbildung beeinflusst die Fluoreszenzlebensdauer des NADH: Die Fluoreszenz des freien NADH ist kurzlebig, weil Moleküle in der umgebenden Flüssigkeit als Quencher wirken. In dem Komplex ist das NADH-Molekül gegen die Wirkung der Quencher weitgehend geschützt und die Fluoreszenz erheblich langlebiger.

Diese Tatsachen sind aus der Literatur bekannt. Völlig unerwartet war hingegen, dass dieser Effekt unter den in praktischen Tests vorherrschenden Bedingung so ausgeprägt ist, dass die in der Praxis auftretenden Änderungen der Analytkonzentration in Blutproben zu einer gut messbaren Verschiebung der mittleren Fluoreszenzlebensdauer führen. Aufgrund der Literaturangaben, insbesondere in dem Dokument (3), wäre zu erwarten gewesen, dass der Bindungsgrad des Komplexes NADH/GlucDH sehr hoch ist. Das von dem Komplex NADH/GlucDH emittierte Fluoreszenzlicht hat eine sehr viel größere Intensität als die Fluoreszenz von freier NADH. Deswegen wäre zu erwarten gewesen, dass die beobachtete Fluoreszenzlebensdauer nur in sehr geringem Ausmaß von der freien NADH abhängig und der mit Konzentrationsänderungen im physiologischen Bereich verbundene Effekt hinsichtlich der Lebensdauer unmessbar gering ist. Im Rahmen der Erfindung wurde jedoch festgestellt, dass unter Praxisbedingungen ein sehr hoher Anteil des NADH (über 90%) unkomplexiert vorliegt.

Die Fluoreszenzlebensdauer liefert zusätzlich zu der Fluoreszenz-intensität eine zweite, unabhängige Information über die Fluoreszenz der Reaktionsprodukte. Sie kann selbständig (also ohne andere Informationen) zur Ermittlung der gesuchten Analytkonzentration verwendet werden. Vorzugsweise wird sie jedoch in Kombination mit der Messung der Fluoreszenzintensität eingesetzt. Dies erfordert keinen zusätzlichen messtechnischen Aufwand, weil übliche Verfahren zur Messung der Fluoreszenzlebensdauer zugleich Messergebnisse über die Fluoreszenzintensität liefern.

Um die Fluoreszenzlebensdauer erfindungsgemäß bei der Bestimmung der gesuchten Analytkonzentration zu verwenden, kann es zweckmäßig sein, sie zu messen und die aus der Messung resultierenden Messwerte in geeigneter Weise innerhalb des Auswertealgorithmus zu berücksichtigen. Eine solche Messung (im strengen Sinne, d. h. Messung von zuvor unbekannten Messwerten) ist jedoch nicht notwendig. Vielmehr kann die Fluoreszenzlebensdauer auch in einer Weise verwendet werden, bei der keine Messung in dem Sinn notwendig ist, dass (zuvor unbekannte) Messwerte der Fluoreszenzlebensdauer bestimmt werden. Eine Möglichkeit besteht darin, die Fluoreszenzintensität innerhalb eines definierten Zeitfensters zu messen, wobei das Zeitfenster an die (bekannte) Fluoreszenzlebensdauer eines aus der Reaktion der Probe mit dem Reagenzsystem resultierenden Fluorophors angepasst ist. Der aus einer solchen Messung resultierende Messwert steht in Beziehung zu der Fluoreszenzlebensdauer und wird nachfolgend auch als Lebensdauerbezogene Fluoreszenzintensität bezeichnet. Da er in dem Auswerte-algorithmus verwendet wird, kann auch in diesem Fall davon gesprochen werden, dass die Fluoreszenzlebensdauer in dem Auswertealgorithmus verwendet wird.

Da demzufolge eine Messung der Fluoreszenzlebensdauer (in dem erläuterten strengen Sinn) nicht unbedingt notwendig ist, muss das Auswertegerät eines entsprechenden Analysesystems auch nicht notwendigerweise eine Messeinheit aufweisen, die dazu ausgebildet ist, die Fluoreszenzlebensdauer des Fluorophors zu messen. Vielmehr besteht alternativ oder zusätzlich die Möglichkeit, das die Messeinheit des Auswertegeräts so ausgebildet ist, dass sie die Fluoreszenzlebensdauer in anderer Weise, beispielsweise mittels des erwähnten bei der Intensitätsmessung gesetzten Zeitfensters, berücksichtigt.

Geeignete Verfahren zur Messung der Fluoreszenzlebensdauer sind bekannt. Grundsätzlich für die Erfindung geeignet ist eine zeitaufgelöste Fluoreszenzdetektion, bei der ein sehr kurzer Puls des Anregungslichts eingestrahlt und der Verlauf der resultierenden Emissionskurve mit geeigneten zeitlich hochauflösenden Detektionsverfahren detektiert wird. Vorzugsweise wird im Rahmen der Erfindung jedoch ein mit kontinuierlicher Einstrahlung arbeitendes Phasenmodulationsverfahren verwendet. Nähere Informationen über geeignete Verfahren können der Literatur entnommen werden. Zu verweisen ist insbesondere auf das als Dokument (4) zitierte Buch von Lakovicz sowie folgende weitere Publikationen und die darin zitierte Literatur:

(5) T. G. Scott et al. "Emission Properties of NADH. Studies of Fluorescence Lifetimes ..."; J. Am. Chem. Soc., 1970, 687 - 695
(6) US-patent 5,485,530
(7) EP 0 561 653 A1

Durch die Erfindung werden u.a. folgende Vorteile erreicht:

- Die oben angesprochenen Probleme werden weitgehend vermieden. Insbesondere ist keine Kalibration der Fluoreszenzintensität mittels eines Standard-Fluorophors notwendig.

- Die hochfrequente Messung führt zu einer Reduktion des Einflusses von störendem Umlicht.

- Die Erfindung ermöglicht eine erhöhte Genauigkeit, insbesondere durch Elimination oder Reduzierung von Fehlerquellen.

Die Erfindung wird nachfolgend anhand der Figuren näher erläutert. Die dargestellten und beschriebenen Besonderheiten der Erfindung können einzeln oder in Kombination verwendet werden, um bevorzugte Ausführungsformen der Erfindung zu schaffen. Es zeigen:

Fig. 1    eine Prinzipskizze der wesentlichen Funktionskomponenten eines für die Erfindung geeigneten Analysesystems;

Fig. 2    Messergebnisse hinsichtlich der mittleren Lebensdauer der Fluoreszenz in Abhängigkeit von dem Konzentrationsverhältnis zwischen GlucDH und NADH;

Fig. 3    Messergebnis hinsichtlich des funktionalen Zusammenhangs zwischen der Glucosekonzentration und dem Intensitätsverhältnis zwischen kurzlebiger und langlebiger Fluoreszenz;

Fig. 4    einen Vergleich der erfindungsgemäß gemessenen Glucosekonzentration mit den Ergebnissen eines Referenzmessverfahrens.

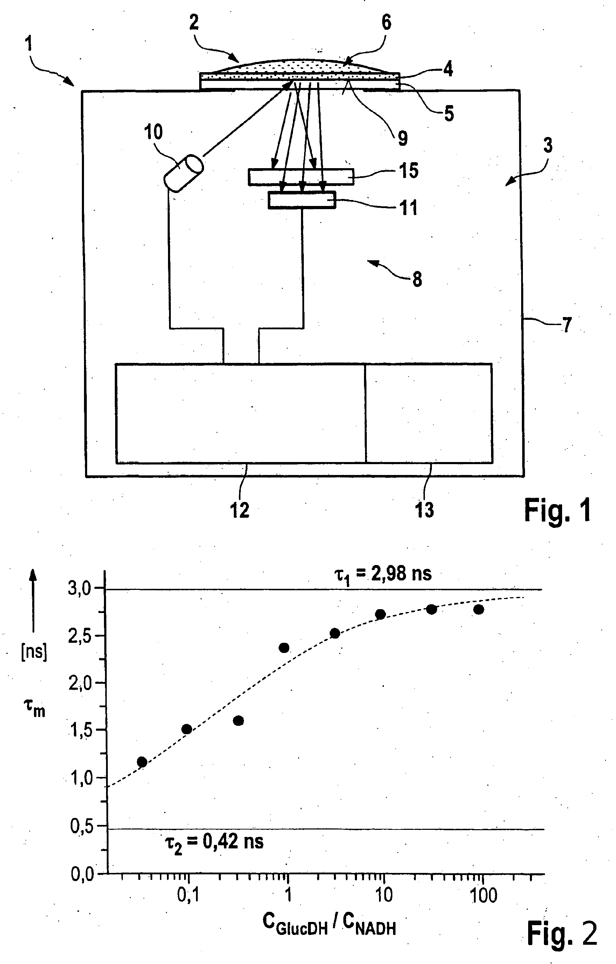

Das in Figur 1 stark schematisiert dargestellte Analysesystem besteht aus zwei aufeinander abgestimmten Komponenten, nämlich einem Analyseelement 2 und einem Auswertegerät 3. Das Analyseelement 2 umfasst eine Reagenzschicht 4, die ein Reagenzsystem in einer geeigneten Matrix, beispielsweise einer Gelmatrix, enthält. Die Reagenzschicht 4 befindet sich auf einem optisch transparenten Träger 5. Die Reagenzien des Reagenzsystems sind in die Matrix der Reagenzschicht 4 in trockener Form eingebettet. Wenn eine Probenflüssigkeit 6 auf die freie Oberfläche der Reagenzschicht 4 aufgegeben wird und in diese eindringt, werden die Reagenzien des Reagenzsystems gelöst und es findet eine Reaktion der Probenflüssigkeit statt, die zu einer Änderung der Menge eines Fluorophors führt, welcher ein Bestandteil des Reagenzsystems ist. Der Aufbau solcher Analyseelemente wird beispielsweise in den Dokumenten US 3,802,842; US 4,061,468; US 2006/0003397 beschrieben.

Das Gehäuse 7 des Auswertegerätes 3 enthält eine insgesamt mit 8 bezeichnete Messeinheit, die geeignet ist, eine für die Menge des Fluorophors charakteristische Messgröße zu bestimmen. Die Messeinheit 8 schließt eine Anregungslichtquelle 10 ein, beispielsweise einen Laser oder eine LED, deren Licht von hinten (durch den optisch transparenten Träger 5) auf die von der Probenaufgabeseite abgewandte, dem optisch transparenten Träger 5 zugewandte Oberfläche der Reagenzschicht 4 gerichtet ist. Dabei wird von der Auswertezone 9 Sekundärlicht in Richtung auf einen Detektor 11 abgestrahlt und von diesem detektiert. Das resultierende Messsignal wird einer Signalverarbeitungseinheit zugeführt und in üblicher Weise verstärkt, aufbereitet und digitalisiert. Die resultierenden digitalisierten Messwerte werden einer Auswerteeinheit 13 zugeführt, die die erforderliche Soft- und Hardware enthält, um aus den digitalisierten Messwerten die gesuchte Konzentration des Analyten zu ermitteln.

Die Messeinheit 8 ist geeignet, von der Auswertezone 9 abgestrahltes Fluoreszenzlicht zu detektieren. Zu diesem Zweck wird mit üblichen Mitteln, beispielsweise einem in Figur 1 dargestellten Filterelement 15 der Zutritt des von der Anregungslichtquelle 10 ausgehenden Primärlichtes derartig blockiert, dass das mit einer höheren Wellenlänge abgestrahlte Fluoreszenzlicht selektiv von dem Detektor 11 erfasst werden kann. Insoweit ist das erfindungsgemäße Analysesystem konventionell aufgebaut und muss deshalb nicht näher erläutert werden. Nähere Informationen können beispielsweise den zitierten Dokumenten des Standes der Technik entnommen werden.

Eine Besonderheit des in Figur 1 dargestellten erfindungsgemäßen Analysesystems besteht darin, dass die Messeinheit 8 dazu geeignet ist, die mittlere Fluoreszenzlebensdauer zu messen und/oder (im Rahmen der Messung einer anderen Messgröße) zu berücksichtigen. Wie bereits erwähnt, erfolgt die Fluoreszenzlebensdauer-Messung vorzugsweise mit einem Phasenmodulationsverfahren. Dabei wird das von der Anregungslichtquelle 10 abgestrahlte Licht hochfrequent moduliert und die gesuchte Information über die Fluoreszenzlebensdauer aus der mittels des Detektors 11 und der Signalverarbeitungseinheit 12 gemessenen Phasenverschiebung ermittelt. Auch diesbezüglich ist eine nähere Erläuterung nicht erforderlich, weil ergänzende Informationen der Literatur, insbesondere den oben erwähnten Dokumenten, entnommen werden können.

Figur 2 zeigt experimentelle Ergebnisse aus der der Erfindung zugrundeliegenden Forschungsarbeit. Dargestellt ist die mittlere Fluoreszenzlebensdauer $\tau_m$ für verschiedene Konzentrationsverhältnisse einer flüssigen Mischung von GlucDH und NADH. Einer NADH-Lösung mit einer Konzentration von 1,2 mg/l wurden variierende Mengen von GlucDH zugemischt, um die auf der Abszisse angegebenen Konzentrationen einzustellen. Figur 2 zeigt, dass die mit der Variation der Konzentration von GlucDH verbundene Verschiebung des Verhältnisses zwischen freier und komplexierter NADH durch Lebensdauermessungen gut beobachtet werden kann, wobei sich die Messkurve für sehr hohe und sehr niedrige GlucDH-Korizentrationen den Extremwerten $\tau_1 = 2,98$ ns und $\tau_2 = 0,42$ ns nähert. Da der Komplexierungsgrad von der Menge der fluoreszierenden Spezies abhängt, ist auch die mittlere Fluoreszenzlebensdauer eine Messgröße, die für die Menge des Fluorophors charakteristisch ist.

Aus den in Figur 2 dargestellten Messergebnissen wurde im Rahmen der Erfindung abgeleitet, dass die Änderung der mittleren Lebensdauer als Information zur Quantifizierung eines Analyten in Fluoreszenztests verwendet werden kann. Diese Erkenntnis kann auf unterschiedliche Weise analytisch genutzt werden.

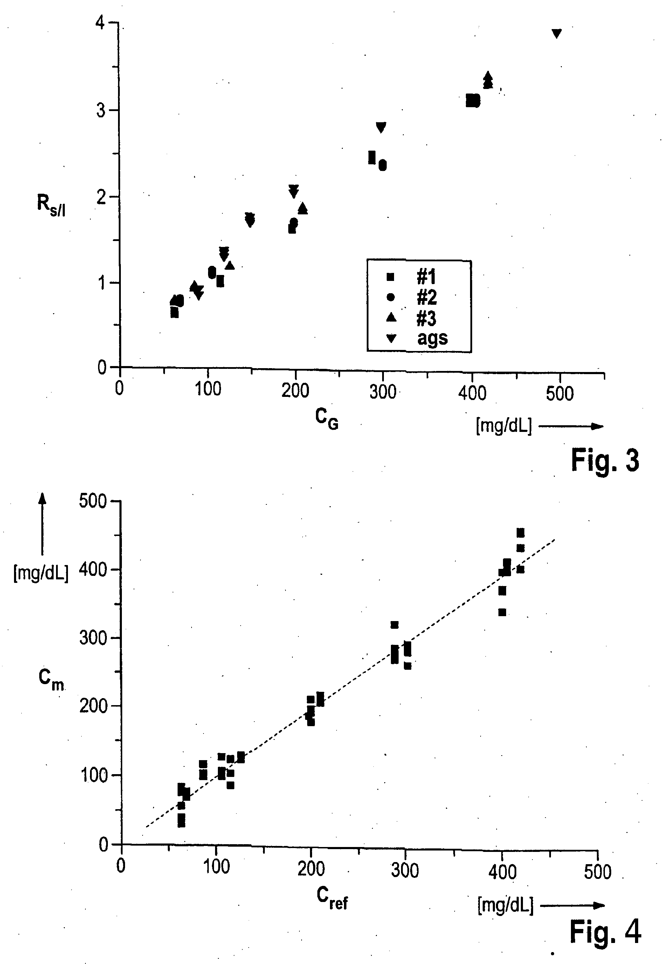

Figur 3 verdeutlicht eine bevorzugte Möglichkeit, bei der die Messung der Fluoreszenzlebensdauer dazu verwendet wird, eine Lebensdauer-bezogene Fluoreszenzintensität (Lifetime Related Fluorescence Intensity; LRFI) zu bestimmen. Ein solcher LRFI-Wert beschreibt die Intensität der Fluoreszenz in Abhängigkeit von der Fluoreszenzlebensdauer und bildet somit ein Maß für die Mengenrelation von fluoreszierenden Spezies, die sich durch unterschiedliche mittlere Lebensdauern auszeichnen, wie beispielsweise komplexiertes NADH im Verhältnis zu freiem NADH. Auf der Basis von durch kurze Lichtimpulse angeregten zeitlich hochaufgelöst gemessenen Fluoreszenzsignalen kann man einen LRFI-Wert beispielsweise dadurch ermitteln, dass der Abfall ("decay") des Fluoreszenzsignals eine logarithmische Funktion ist, die von den mittleren Fluoreszenzlebensdauern der fluoreszierenden Spezies und deren Konzentration abhängt. Die getrennten Intensitäten der beiden Signalkomponenten können durch mathematisches Fitten solcher Messkurven ermittelt werden.

Figur 3 zeigt die Abhängigkeit solcher LRFI-Werte von der Glucosekonzentration für einen Glucose-Fluoreszenztest mit Analyseelementen, wie sie in den oben genannten Dokumenten (2) und (3) beschrieben sind. Dargestellt ist ein Verhältnis $R_{s/l}$ zwischen dem LRFI-Wert für kurzlebiges freies NADH und dem LRFI-Wert für langlebiges komplexiertes NADH. Die Messungen wurden mit Blutproben unterschiedlicher Hämatokrit-Werte und mit einer wässrigen Glucoselösung durchgeführt, wobei die resultierenden Messwerte in Figur 3 mit unterschiedlichen Symbolen bezeichnet sind. Darin bezeichnen #1 bis #3 die Nummern von Proben mit unterschiedlichen Hämatokritkonzentrationen (sehr hoch - Normalbereich - sehr niedrig); "ags" bedeutet "aqueous glucose solution". Man erkennt eine erstaunlich gute Korrelation zwischen der Glucosekonzentration und dem $R_{sl}$-Wert, wobei die Messwerte weitgehend unabhängig sind von dem Hämatokrit-Wert, also von der Konzentration der Blutkörperchen in der Probe.

Figur 3 zeigt ein erstes Beispiel einer bevorzugten Ausführungsform der Erfindung, bei der zwei LRFI-Werte in Beziehung zueinander gesetzt werden. Dabei wurden $R_{s/l}$ berechnet gemäß

$$R_{s/l} = \frac{A_2 \cdot \tau_2}{A_1 \cdot \tau_1}$$

wobei A die Amplitude, also ein Maß der Intensität und $\tau$ die Fluoreszenzlebensdauer der beiden mit unterschiedlicher Lebensdauer fluoreszierenden Spezies bezeichnet. Der Wert $R_{s/l}$ wird gegen die Analytkonzentration kalibriert und dann in dem Auswertealgorithmus als Maß für die Analytkonzentration verwendet.

Es gibt zahlreiche andere Möglichkeiten, LRFI-Werte derartig zueinander in Beziehung zu setzen, dass eine Rechengröße gebildet wird, die mittels einer Kalibration als Maß für die gesuchte Analytkonzentration verwendet werden kann. Beispielsweise kann eine gemittelte Fluoreszenzlebensdauer $\tau_m$ berechnet werden gemäß

$$\tau_m = \frac{A_1\tau_1 + A_2\tau_2}{A_1 + A_2}$$

Gemäß einer besonders bevorzugten Ausführungsform erfolgt eine flächengewichtete Mittlung gemäß

$$\tau_m = \frac{A_1\tau_1^{\ 2} + A_2\tau_2^{\ 2}}{A_1\tau_1 + A_2\tau_2}$$

Allen diesen Beispielen ist gemeinsam, dass die Fluoreszenzlebensdauern und die Fluoreszenzintensitäten zweier fluoreszierender Spezies als für die Analyse charakteristische Messgrößen verwendet werden. Sie werden jeweils gemäß einer vordefinierten mathematischen Gleichung in Beziehung zueinander gesetzt, um eine Rechengröße zu ermitteln, die gegen die gesuchte Analytkonzentration kalibriert werden kann. Die Verwendung von zwei simultan gemessenen Messwerten und die Bildung eines Quotienten führt dazu, dass Messfehler, die beide Fluoreszenzmesswerte in gleicher Weise beeinflussen, beispielsweise durch Verunreinigungen oder kleine Beschädigungen im optischen Messkanal, nicht oder nur in sehr viel geringerem Umfang das Messergebnis negativ beeinflussen, als bei vorbekannten Messungen der Fluoreszenzintensität.

Die Messung von LRFI-Werten ist ein Beispiel dafür, dass im Rahmen der Erfindung nicht notwendigerweise eine Messung einer Fluoreszenzlebensdauer (im strengen Sinne) notwendig ist. Wie oben dargelegt, kann die Messeinrichtung des Auswertegerätes auch so ausgebildet sein, dass sie eine Fluoreszenzintensität eines definiert gesetzten Zeitfensters erfasst und damit ein LRFI-Wert gemessen wird, wobei vorzugsweise zwei oder mehrere derartige LRFI-Werte, ähnlich wie in den vorstehenden Beispielen erläutert, in Beziehung zueinander gesetzt werden, um eine Rechengröße zu ermitteln, die gegen die Analytkonzentration geeicht werden kann.

Figur 4 zeigt die Korrelation von erfindungsgemäß ermittelten Glucosekonzentrationswerten $C_m$ mit Glucosewerten $C_{ref}$, die mit einer Referenzmethode ermittelt wurden. Auch hier zeigt sich, dass die auf der Grundlage der mittleren Fluoreszenzlebensdauer ermittelten Analysewerte erstaunlich gut mit den Werten der Referenzmethode übereinstimmen.

Der Fluorphor ist ein Coenzym, ausgewählt aus der Gruppe bestehend aus NADH/H⁺ und NADPH/H⁺, einschließlich deren Derivaten. Geeignet sind insbesondere Derivate, bei denen der im wesentlichen für die Fluoreszenz verantwortliche Molekülteil unverändert bleibt. Bei solchen Derivaten sind grundsätzlich analoge Fluoreszenzeigenschaften zu erwarten, wobei sich natürlich die Parameter der Fluoreszenz, wie z.B. die Wellenlänge der Absorption oder Emission ändern können. Da bei NAD/NADH im wesentlichen der Pyridinring für die Fluoreszenz verantwortlich ist, sind insbesondere Derivate geeignet, bei denen der Pyridinring nicht modifiziert ist. Für die vorliegende Erfindung geeignete Derivate sind beispielsweise in der WO2007/012494 und der US2007/26476 beschrieben. Insbesondere eignet sich zum Einsatz für das erfindungsgemäße Verfahren carbaNAD, ein Derivat ohne Glycosyl-Bindung, welches schon 1988 beschrieben wurde (J.T. Slama, Biochemistry 1989, 27, 183 und Biochemistry 1989, 28, 7688). Darin wird die Ribose durch eine carbazyklische Zuckereinheit substituiert. Selbstverständlich sind nicht alle Derivate von NADH/H⁺ und NADPH/H⁺ gleichermaßen für die Erfindung geeignet. Die Eignung für den erfindungsgemäßen Einsatz kann jedoch problemlos experimentell geprüft werden.

Das Enzym eines im Rahmen der Erfindung verwendeten Enzym/Coenzym-Paares ist Glucose-Dehydrogenase (E.C.1.1.1.47). z.B. L-Aminosäure-Dehydrogenase.

Wie beschrieben korreliert die mittlere Fluoreszenzlebensdauer so gut mit der Konzentration des Analyten, dass sie selbständig als für die Konzentration charakteristische Messgröße in dem Auswertealgorithmus für die Berechnung der Analytkonzentration verwendet werden kann. Vorzugsweise wird sie jedoch in Kombination mit einer anderen die Konzentration charakterisierenden Messgröße, insbesondere der Fluoreszenzintensität, aber auch der optischen Absorption verwendet. Dabei kann sie auch zur Anwendung kommen, um Messfehler zu kompensieren, die durch Störgrößen, insbesondere die Temperatur der Probe oder deren Hämatokrit, verursacht werden.

## Patentansprüche

**1.** Verfahren zur Bestimmung der Konzentration von Glucose in einer flüssigen Probe mittels eines Reagenzsystems, das ein Enzym/Coenzym-Paar mit Glucosedehydrogenase (GlucDH) als Enzym und NAD und/oder NADP einschließlich deren geeigneten Derivate, bei. denen der im Wesentlichen für die Fluoreszenz verantwortliche Molekülteil unverändert ist, als Coenzym enthält, wobei
die Reaktion der Probe mit dem Reagenzsystem zu einer Änderung der Menge eines Fluorophors führt, der ausgewählt ist aus der Gruppe bestehend aus NADH/H⁺ und NADPH/H', einschließlich deren geeigneten Derivate, bei denen der im Wesentlichen für die Fluoreszenz verantwortliche Molekülteil unverändert ist,
die Menge des Fluorophors mit der Konzentration der Glucose korreliert,
eine für die Menge des Fluorophors charakteristische Messgröße gemessen und
die Konzentration der Glucose mittels eines Auswertealgorithmus auf Basis der Messgröße ermittelt wird,

**dadurch gekennzeichnet, dass**
das Reagenzsystem in einem Analyseelement in getrockneter Form integriert ist,
die Reaktion der Probe mit dem Reagenzsystem eine Komplexbildung unter Beteiligung eines Enzym/Coenzym-Paares einschließt, wobei das Enzym GlucDH und das Coenzym der Fluorophor ist, und
die mit der Komplexbildung verbundene Änderung der mittleren Fluoreszenzlebensdauer des Fluorophors bei der Ermittlung der Konzentration der Glucose verwendet wird.

2. Verfahren nach Anspruch 1, **dadurch gekennzeichnet, dass** der Fluorophor ein Derivat des NADH/H$^+$ oder des NADPH/H$^+$, dessen Pyridinring nicht modifiziert ist, oder carbaNAD ist.

3. Verfahren nach einem der vorhergehenden Ansprüche, **dadurch gekennzeichnet, dass** die gemessene mittlere Fluoreszenzlebensdauer des Fluorophors in dem Auswertealgorithmus als eine für die Konzentration charakteristische Messgröße verwendet wird.

4. Verfahren nach einem der vorhergehenden Ansprüche, **dadurch gekennzeichnet, dass** die gemessene mittlere Fluoreszenzlebensdauer in dem Auswertealgorithmus in Kombination mit einer anderen für die Konzentration charakteristischen Messgröße, insbesondere der Fluoreszenzintensität, verwendet wird.

5. Verfahren nach Anspruch 4, **dadurch gekennzeichnet, dass** die gemessener mittlere Fluoreszenzlebensdauer in dem Auswertealgorithmus zur Kompensation von Messfehlern verwendet wird, die durch Störgrößen verursacht werden.

6. Verfahren nach einem der vorhergehenden Ansprüche, **dadurch gekennzeichnet, dass** die Messung der mittleren Fluoreszenzlebensdauer verwendet wird, um einen Lebensdauer-bezogenen Fluoreszenzintensitätswert zu bestimmen.

7. Verfahren nach Anspruch 6, **dadurch gekennzeichnet, dass** die Fluoreszenzintensität für mindestens zwei verschiedene mittlere Fluoreszenzlebensdauern gemessen wird, um jeweils Lebensdauer-bezogene Fluoreszenzintensitätswerte zu bestimmen.

8. Verfahren nach Anspruch 7, **dadurch gekennzeichnet, dass** die beiden Lebensdauer-bezogenen Fluoreszenzintensitätswerte in Beziehung zueinander gesetzt werden.

9. Analysesystem zur Bestimmung der Konzentration von Glucose in einer flüssigen Probe mittels eines Verfahrens nach einem der vorhergehenden Ansprüche,
mit
einem Reagenzsystem, das Glucosedehydrogenase (GlucDH) als Enzym und NAD und/oder NADP einschließlich deren geeigneten Derivate, bei denen der im Wesentlichen für die Fluoreszenz verantwortliche Molekülteil unverändert ist, als Coenzym enthält,
dessen Reaktion mit der Probe zu einer Änderung der Menge eines Fluorophors führt,

- welcher ausgewählt ist aus der Gruppe bestehend aus NADH/H$^+$ und NADPH/H$^+$, einschließlich deren geeigneten Derivate, bei denen der im Wesentlichen für die Fluoreszenz verantwortliche Molekülteil unverändert ist,
- wobei die Menge des Fluorophors mit der Konzentration der Glucose korreliert,
- wobei die Reaktion der Probe mit dem Reagenzsystem eine Komplexbildung unter Beteiligung eines Enzym/Coenzym-Paares einschließt, wobei das Enzym GlucDH und das Coenzym der Fluorophor ist, und

einem Auswertegerät, welches eine Messeinheit zur Messung einer für die Menge des Fluorophors charakteristischen Messgröße, einschließt und welches eine Auswerteeinheit einschließt, um mittels eines Auswertealgorithmus auf der Basis der Messgröße die Konzentration der Glucose zu ermitteln,
**dadurch gekennzeichnet, dass**
die Reagenzien des Reagenzsystems in trockener Form in einem Analyseelement enthalten sind, und
die Messeinheit des Auswertegerätes dazu ausgebildet ist, eine mit der Komplexbildung verbundene Änderung der mittleren Fluoreszenzlebensdauer des Fluorophors zu messen und/oder zu berücksichtigen.

**Claims**

1. Method for determining the concentration of glucose in a liquid sample using a reagent system, which contains an enzyme/coenzyme pair with glucose dehydrogenase (GlucDH) acting as enzyme and NAD and/or NADP including their suitable derivates, in which the molecule part that is essentially responsible for fluorescence remains unchanged, acting as coenzyme, wherein
the reaction of the sample with the reagent system results in a change of the quantity of a fluorophore, the fluorophore being selected from the group comprising NADH/H$^+$ and NADPH/H$^+$, including their suitable derivates, in which the molecule part that is essentially responsible for fluorescence remains unchanged,
the quantity of the fluorophore correlates with the concentration of glucose,
a measurement variable that is characteristic for the quantity of the fluorophore is measured, and
the concentration of glucose is determined on the basis of the measurement variable using an evaluation algorithm, **characterized in that**
the reagent system is integrated in dry form in an analysis element, the reaction of the sample with the reagent system includes a complex formation with participation of an enzyme/coenzyme pair, wherein the enzyme is GlucDH and the coenzyme is the fluorophore, and
the change in the medium fluorescence lifetime of the fluorophore associated with the complex formation is used in the determination of the concentration of glucose.

2. Method according to claim 1, **characterized in that** the fluorophore is a derivative of NADH/H$^+$ or NADPH/H$^+$, whose pyridine ring is not modified, or is carbaNAD.

3. Method according to any one of the preceding claims, **characterized in that** the measured medium fluorescence lifetime of the fluorophore is used in the evaluation algorithm as a measurement variable that is characteristic for the concentration.

4. Method according to any one of the preceding claims, **characterized in that** the measured medium fluorescence lifetime is used in the evaluation algorithm in combination with another measurement variable that is characteristic for the concentration, in particular the fluorescence intensity.

5. Method according to claim 4, **characterized in that** the measured medium fluorescence lifetime is used in the evaluation algorithm to compensate for measurement errors that are caused by interfering variables.

6. Method according to any one of the preceding claims, **characterized in that** the measurement of the medium fluorescence lifetime is used to determine a lifetime-related value of the fluorescence intensity.

7. Method according to claim 6, **characterized in that** the fluorescence intensity is measured for at least two different medium fluorescence lifetimes in order to determine in each case lifetime-related values of the fluorescence intensity.

8. Method according to claim 7, **characterized in that** the two lifetime-related values of the fluorescence intensity are related to one another.

9. Analysis system for determining the concentration of glucose in a liquid sample using a method according to any one of the preceding claims,
the system comprising
a reagent system containing glucose dehydrogenase (GlucDH) acting as enzyme and NAD and/or NADP including their suitable derivates, in which the molecule part that is essentially responsible for fluorescence remains unchanged, acting as coenzyme,
whose reaction with the sample results in a change of the quantity of a fluorophore,

- the fluorophore being selected from the group comprising NADH/H$^+$ and NADPH/H$^+$, including their suitable derivates, in which the molecule part that is essentially responsible for fluorescence remains unchanged,
- wherein the quantity of fluorophore correlates with the concentration of glucose,
- wherein the reaction of the sample with the reagent system includes a complex formation with participation of an enzyme/coenzyme pair, wherein GlucDH is the enzyme and the fluorophore is the coenzyme, and

an evaluation instrument comprising a measuring unit for measuring a measurement variable that is characteristic for the quantity of the fluorophore, and an evaluation unit for determining the concentration of glucose on the basis of the measurement variable, using an evaluation algorithm,
**characterized in that**
the reagents of the reagent system are contained in dry form in an analysis element, and
the measuring unit of the analysis instrument is adapted for measuring and/or taking into account a change in the medium fluorescence lifetime of the fluorophore associated with the complex formation.

## Revendications

1. Procédé de détermination de la concentration de glucose dans un échantillon liquide au moyen d'un système de réactifs qui contient une paire enzyme/coenzyme avec une glucose déshydrogénase (GlucDH) en tant qu'enzyme et de la NAD et/ou du NADP, y compris leurs dérivés appropriés pour lesquels la part moléculaire essentiellement responsable de la fluorescence n'est pas modifiée, en tant que coenzyme,
dans lequel,
la réaction de l'échantillon avec le système de réactifs conduit à une modification de la quantité d'un fluorophore qui est sélectionné parmi le groupe constitué de NADH/H$^+$ et NADPH/H$^+$, y compris leurs dérivés appropriés pour lesquels la part moléculaire essentiellement responsable de la fluorescence n'est pas modifiée,
la quantité du fluorophore est corrélée à la concentration du glucose,
une valeur caractéristique de la quantité du fluorophore est mesurée et
la concentration du glucose est déterminée au moyen d'un algorithme d'évaluation sur la base de ladite valeur,
**caractérisé en ce que**
le système de réactifs est intégré dans un élément d'analyse sous une forme sèche,
la réaction de l'échantillon avec le système de réactifs inclut une formation de complexes avec participation d'une paire enzyme/coenzyme, dans lequel l'enzyme est la GlucDH et le coenzyme est le fluorophore, et
la modification, liée à la formation de complexes, de la durée de vie moyenne de fluorescence du fluorophore est utilisée lors de la détermination de la concentration du glucose.

2. Procédé selon la revendication 1, **caractérisé en ce que** le fluorophore est un dérivé du NADH/H$^+$ ou du NADPH/H$^+$, dont le cycle pyridine n'est pas modifié, ou est une carbaNAD.

3. Procédé selon l'une quelconque des revendications précédentes, **caractérisé en ce que** la durée de vie moyenne de fluorescence mesurée du fluorophore est utilisée dans l'algorithme d'évaluation en tant que valeur caractéristique de la concentration.

4. Procédé selon l'une quelconque des revendications précédentes, **caractérisé en ce que** la durée de vie moyenne de fluorescence mesurée est utilisée dans l'algorithme d'évaluation en combinaison avec une autre valeur caractéristique de la concentration, en particulier l'intensité de fluorescence.

5. Procédé selon la revendication 4, **caractérisé en ce que** la durée de vie moyenne de fluorescence mesurée est utilisée dans l'algorithme d'évaluation pour compenser des erreurs de mesure provoquées par des perturbations.

6. Procédé selon l'une quelconque des revendications précédentes, **caractérisé en ce que** la mesure de la durée de vie moyenne de fluorescence est utilisée pour déterminer une valeur d'intensité de fluorescence en lien avec la durée de vie.

7. Procédé selon la revendication 6, **caractérisé en ce que** l'intensité de fluorescence est mesurée pour au moins deux durées de vie moyennes de fluorescence différentes, afin de déterminer respectivement des valeurs d'intensité de fluorescence en lien avec la durée de vie.

8. Procédé selon la revendication 7, **caractérisé en ce que** les deux valeurs d'intensité de fluorescence en lien avec la durée de vie sont mises en relation l'une par rapport à l'autre.

9. Système d'analyse destiné à déterminer la concentration de glucose dans un échantillon liquide au moyen d'un procédé selon l'une quelconque des revendications précédentes,
avec
un système de réactifs qui contient une glucose déshydrogénase (GlucDH) en tant qu'enzyme et de la NAD et/ou du NADP, y compris leurs dérivés appropriés pour lesquels la part moléculaire essentiellement responsable de la fluorescence n'est pas modifiée, en tant que coenzyme,

dont la réaction avec l'échantillon conduit à une modification de la quantité d'un fluorophore,

- qui est sélectionné parmi le groupe constitué de NADH/H$^+$ et NADPH/H$^+$, y compris leurs dérivés appropriés pour lesquels la part moléculaire essentiellement responsable de la fluorescence n'est pas modifiée,
- dans lequel la quantité du fluorophore est corrélée à la concentration du glucose,
- dans lequel la réaction de l'échantillon avec le système de réactifs inclut une formation de complexes avec participation d'une paire enzyme/coenzyme, dans lequel l'enzyme est la GlucDH et le coenzyme est le fluorophore, et

un appareil d'évaluation, qui inclut une unité de mesure destinée à mesurer une valeur caractéristique de la quantité du fluorophore et qui inclut une unité d'évaluation pour déterminer la concentration du glucose au moyen d'un algorithme d'évaluation sur la base de ladite valeur,
**caractérisé en ce que**
les réactifs du système de réactifs sont contenus sous une forme sèche dans un élément d'analyse, et
l'unité de mesure de l'appareil d'évaluation est conçue pour mesurer et/ou considérer une modification, liée à la formation de complexes, de la durée de vie moyenne de fluorescence du fluorophore.

**Fig. 1**

**Fig. 2**

Fig. 3

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0293732 A2
- US 20050214891 A1
- US 20060003397 A1
- WO 9400602 A
- US 5485530 B
- EP 0561653 A1
- US 3802842 A
- US 4061468 A
- US 20060003397 A
- WO 2007012494 A
- US 200726476 B

### In der Beschreibung aufgeführte Nicht-Patentliteratur

- **J.R. LAKOVICZ.** Principles of Fluorescence Spectroscopy. Springer Science and Business Media, 2006
- **T. G. SCOTT et al.** Emission Properties of NADH. Studies of Fluorescence Lifetimes ... *J. Am. Chem. Soc.,* 1970, 687-695
- **J.T. SLAMA.** *Biochemistry,* 1989, vol. 27, 183
- *Biochemistry,* 1989, vol. 28, 7688